(19) Europäisches Patentamt
European Patent Office
Office européen des brevets

(11) Numéro de publication : 0 409 729 B1

(12) # FASCICULE DE BREVET EUROPEEN

(45) Date de publication du fascicule du brevet : **12.01.94 Bulletin 94/02**

(21) Numéro de dépôt : **90402073.2**

(22) Date de dépôt : **18.07.90**

(51) Int. Cl.$^5$ : **C07C 327/32,** C07C 327/26, C07C 327/28, A61K 31/265, A61K 7/48, A61K 7/06

(54) **Thioesters bi-aromatiques, leur procédé de préparation et leur utilisation en médecine humaine ou vétérinaire et en cosmétique.**

(30) Priorité : **18.07.89 FR 8909651**

(43) Date de publication de la demande : **23.01.91 Bulletin 91/04**

(45) Mention de la délivrance du brevet : **12.01.94 Bulletin 94/02**

(84) Etats contractants désignés : **BE CH DE ES GB GR IT LI NL**

(56) Documents cités :
**EP-A- 0 137 395**
**DE-A- 2 603 293**
**CHEMICAL ABSTRACTS, vol. 91, 1979, page 195, abstract no. 169862x, Columbus, Ohio, US; B.T. WALTON et al.: "Structure-activity relationships for insect growth regulators derived from substituted di-tert-butyl phenols"**
**TETRAHEDRON LETTERS, vol. 30, 1989, no. 44, pages 6089-6090, Pergamon Press Plc., GB; D. RAVI et al.: "A new mild method for the synthesis of esters and benzenethiol esters by activation of pyridine-2-thiol or benzothiazol-2-thiol esters by methyl iodide"**

(73) Titulaire : **CENTRE INTERNATIONAL DE RECHERCHES DERMATOLOGIQUES GALDERMA, ( CIRD GALDERMA)**
**635, Route Des Lucioles, Sophia Antipolis**
**F-06560 Valbonne (FR)**

(72) Inventeur : **Shroot, Braham, Villa 35 Hameaux de Val Bosquet, Chemin de Val Bosquet**
**F-06600 Antibes (FR)**
Inventeur : **Bernardon, Jean-Michel**
**21, Chemin Plan Bergier, Le Rouret**
**F-06650 Nice (FR)**
Inventeur : **Pilgrim, William Robert**
**Les Bois de Valbonne no. 11 2035, route de Biot**
**F-06560 Valbonne (FR)**

(74) Mandataire : **Nony, Michel et al**
**Cabinet NONY & CIE 29, rue Cambacérès**
**F-75008 Paris (FR)**

EP 0 409 729 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux thioesters bi-aromatiques, leur procédé de préparation et leur utilisation en médecine humaine et vétérinaire et en cosmétique.

Ces nouveaux thioesters bi-aromatiques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité antitumorale. En outre, ces dérivés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

L'état de la technique est constitué par les composés décrits dans le EP-A-325.540.

Les thioesters bi-aromatiques selon l'invention peuvent être représentés par la formule générale suivante :

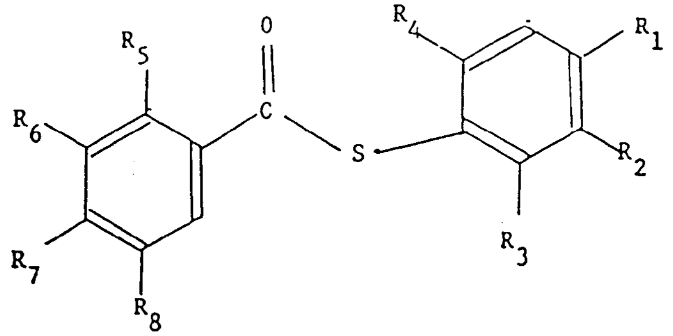

(I)

dans laquelle :

$R_1$ représente un atome d'hydrogène, le groupe OH, le radical $-CH_3$, $-CH_2OH$, $-CH(OH)CH_3$, $-COOR_9$,

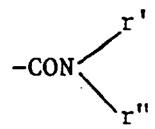

ou $SO_2R_{10}$,

$R_9$ représentant un atome d'hydrogène, un radical alkyle ayant de 1 à 6 atomes de carbone ou un radical mono ou polyhydroxyalkyle,

$R_{10}$représentant le groupe OH, un radical alkyle ayant de 1 à 6 atomes de carbone ou le radical

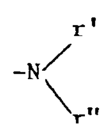

r' et r" représentant un atome d'hydrogène, un radical alkyle ayant de 1 à 6 atomes de carbone, aryle, aralkyle, mono ou polyhydroxyalkyle ou r' et r" pris ensemble forment un hétérocycle,

$R_2$ représente un atome d'hydrogène, un radical alkyle ayant de 1 à 6 atomes de carbone, le radical $OR_9$, un atome de fluor ou le radical $-CF_3$,

$R_3$,$R_4$ et $R_5$ représentant un atome d'hydrogène, un atome de fluor, le groupe OH, le radical $-CH_3$, $-OCH_3$, $-CF_3$,-COOH ou $-CH_2OH$,

$R_5$ et $R_8$ représentent un atome d'hydrogène, le radical $-CF_3$, un radical alkyle $\alpha$ substitué ayant de 3 à 15 atomes de carbone, un radical alkyle $\alpha,\alpha'$-disubstitué ayant de 4 à 12 atomes de carbone, un radical cycloalkyle, ayant de 3 à 12 atomes de carbone, un radical cycloalkyle mono ou polycyclique ayant de 5 à 12 atomes de carbone dont le carbone de liaison est trisubstitué ou le radical $-SR_{11}$, $-SO_2R_{11}$ou $SOR_{11}$,

$R_{11}$ représentant un radical alkyle ayant de 1 à 6 atomes de carbone ou un radical cycloalkyle,

$R_5$ et $R_5$ ne pouvant pas simultanément représenter un atome d'hydrogène,

$R_7$ représente un atome d'hydrogène, un radical alkyle ayant de 1 à 6 atomes de carbone, un radical

alkényle, un radical alkényloxy, le radical $OR_{12}$ ou $SR_{13}$,

$R_{12}$ représentant un atome d'hydrogène, un radical alkyle ayant de 1 à 6 atomes de carbone ou un radical alkényle,

$R_{13}$ représentant un atome d'hydrogène, un radical alkyle ayant de 1 à 6 atomes de carbone ou un radical aralkyle,

$R_6$ et $R_7$, ou $R_7$ et $R_8$ pris ensemble, pouvant également former un cycle à 5 ou 6 chaînons substitué par des groupes méthyle,

sous réserve que lorsque simultanément dans la formule (I) $R_1$ représente $-CH_2OH$, $-CH(OH)CH_3$, $-COOR_9$ ou

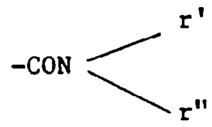

$R_6$ ou $R_6$ représente un radical alkyle α,α'-disubstitué ayant de 4 à 12 atomes de carbone ou un radical cycloalkyle mono ou polycyclique ayant de 5 à 12 atomes de carbone dont le carbone de liaison est trisubstitué,
$R_3$ ou $R_4$ représente un atome d'hydrogène ou le radical méthyle et,
$R_7$ représente le radical $OR_{12}$,
alors au moins un des radicaux $R_2$ ou $R_6$ est différent d'un atome d'hydrogène.

Les composés selon l'invention peuvent également se présenter sous forme de sels lorsqu'ils comportent une fonction acide, il s'agit alors de sels d'un métal alcalin ou alcalino-terreux ou encore de zinc ou d'une amine organique.

Par radical alkyle ayant de 1 à 6 atomes de carbone, on doit entendre de préférence les radicaux méthyle, éthyle, isopropyle, butyle et tertiobutyle.

Par radical alkyle α-substitué ayant de 3 à 15 atomes de carbone on doit entendre un radical isopropyle, 1-méthyl propyle, 1-éthyl propyle, 1-méthyl hexyle, 1- méthyl décyle ou 1-éthyl dodécyle.

Par radical alkyle α,α'-disubstitué ayant de 4 à 12 atomes de carbone on doit notamment entendre un radical tert-butyle, 1,1-diméthyl propyle, 1-méthyl 1-éthyl propyle, 1-méthyl 1-éthyl hexyle ou 1,1-diméthyl décyle.

Par radical monohydroxyalkyle, on doit entendre un radical ayant 2 ou 3 atomes de carbone, notamment un radical 2-hydroxyéthyle, 2-hydroxypropyle ou 3-hydroxypropyle.

Par radical polyhydroxyalkyle, on doit entendre un radical contenant de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical alkényle on doit entendre un radical ayant de 2 à 6 atomes de carbone, tels que les radicaux vinyle, allyle ou 2-butènyle.

Par radical alkényloxy on doit entendre un radical ayant de 2 à 6 atomes de carbone tels que les radicaux vinyloxy, allyloxy ou 2-buténylоxy.

Par radical aryle, on doit entendre un radical phényle éventuellement substitué par au moins un atome d'halogène, un hydroxyle ou une fonction nitro.

Par radical aralkyle on doit entendre le radical benzyle ou phénéthyle éventuellement substitué par au moins un atome d'halogène, un hydroxyle ou une fonction nitro.

Par radical cycloalkyle on doit entendre un radical cyclopentyle ou cyclohexyle.

Par radical cycloalkyle mono ou polycyclique ayant de 5 à 12 atomes de carbone dont le carbone de liaison est trisubstitué on doit entendre le radical 1-méthyl cyclohexyle ou 1-adamantyle.

Lorsque les radicaux r' et r" pris ensemble forment un hétérocycle, celui-ci est de préférence un radical pipéridino, morpholino, pyrrolidino ou pipérazino, éventuellement substitué en position 4 par un radical alkyle en $C_1$-$C_6$ou mono ou polyhydroxyalkyle tels que définis ci-dessus.

Parmi les composés de formule (I) ci-dessus, on peut notamment citer les suivants :

Acide 4-[3-(1-adamantyl)benzoylthio]benzoïque,
Acide 4-[5-(1-adamantyl)-2-fluoro-4-méthoxybenzoylthio]benzoïque,
Acide 4-(5,6,7,8-tétrahydro-5,5,8,8-tétraméthyl-2-naphtoylthio)benzoïque,
Acide 4-[5-(1-adamantyl)-4-méthoxy-2-méthylbenzoylthio]benzoïque,
Acide 4-[5-(1-adamantyl)-2-hydroxy-4-méthoxybenzoylthio]benzoïque,
Acide 4-(3,5-di-trifluorométhylbenzoylthio)benzoïque,
Acide 4-(3-isopropyl-4-méthoxybenzoylthio)benzoïque,

Acide 4-(3-isopropylthio-4-méthylbenzoylthio)benzoïque,
Acide 4-[3-(1-adamantyl)-4-allyloxybenzoylthio]benzoïque,
Acide 4-[3-(1-adamantyl)-4-méthylthiobenzoylthio]benzoïque,
3-(1-adamantyl)-4-méthoxythiobenzoate de phényle
3-(1-adamantyl)-4-méthoxythiobenzoate de 4-hydroxyphényle.

Les composés selon l'invention particulièrement préférés peuvent être représentés par les formules (II) à (IV) suivantes :

(a)

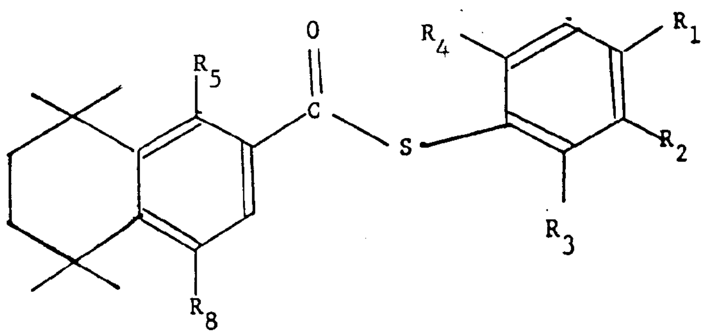

(II)

dans laquelle :

$R_1$ à $R_5$ et $R_8$ sont tels que définis ci-dessus pour la formule (I)

(b)

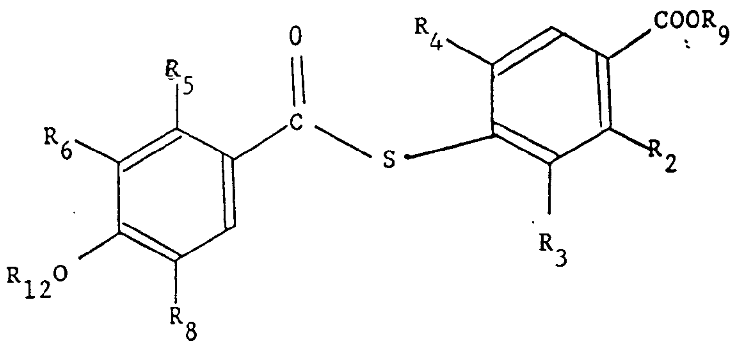

(III)

dans laquelle:

$R_2$ à $R_5$, $R_9$ et $R_{12}$ sont tels que définis ci-dessus pour la formule (I),

$R_6$ et $R_8$ représentent un atome d'hydrogène, un radical alkyle $\alpha,\alpha'$-disubstitué ayant de 4 à 12 atomes de carbone ou un radical cycloalkyle mono ou polycyclique ayant de 5 à 12 atomes de carbone dont le carbone de liaison est trisubstitué,

sous réserve que l'un au moins des radicaux $R_6$ ou $R_8$ est différent d'un atome d'hydrogène et que l'un au moins des radicaux $R_2$, $R_4$ ou $R_6$ est différent d'un atome d'hydrogène

(c)

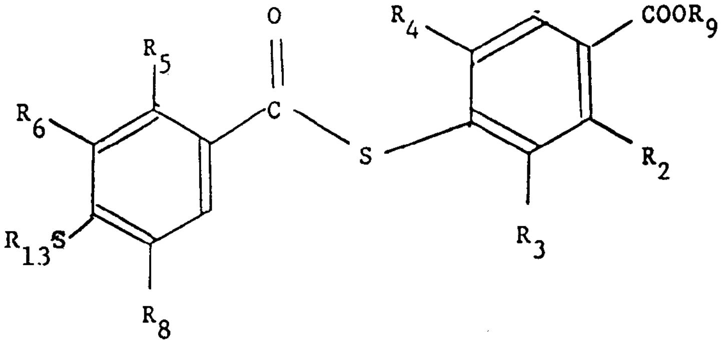

(IV)

dans laquelle :

$R_2$ à $R_6$, $R_8$, $R_9$ et $R_{13}$ sont tels que définis pour la formule (I).

La présente invention a également pour objet le procédé de préparation des composés de formule (I) selon le schéma réactionnel suivant:

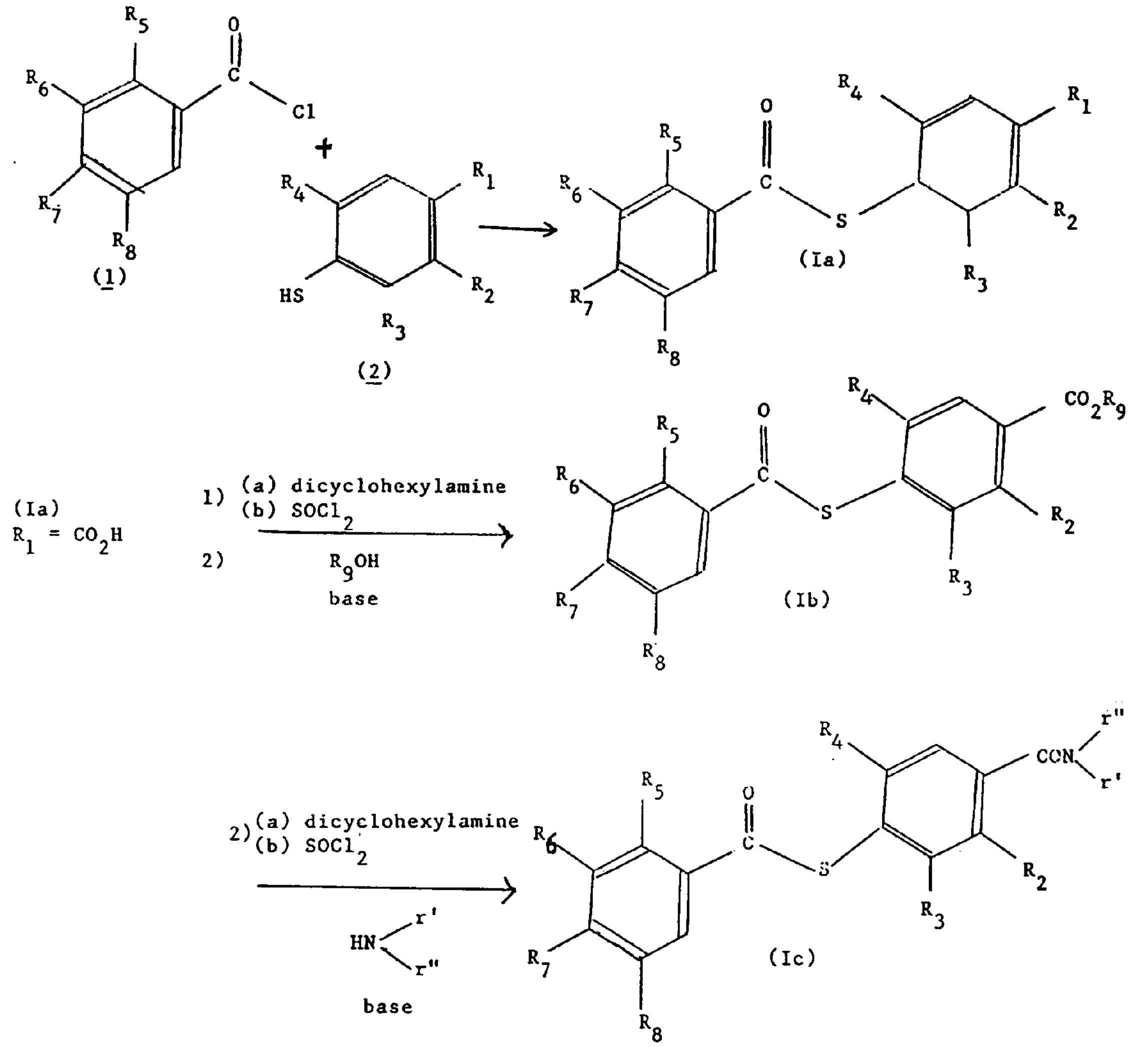


L'étape principale de cette préparation est à rapprocher de celle décrite dans la demande EP 0.137.398 et consiste à faire réagir en milieu anhydre, dans un solvant organique tel que le tétrahydrofuranne ou le chlorure de méthylène contenant une amine tertiaire (pyridine ou triéthylamine), ou un hydrure alcalin tel que l'hydrure de sodium, une forme activée d'un acide benzoïque substitué, par exemple un chlorure d'acide (1), ou un anhydride mixte, sur un benzènethiol (2), la réaction étant conduite à température ambiante et sous agitation.

Lorsque $R_1$= $-CO_2H$, les acides (Ia) ainsi obtenus peuvent être convertis de manière connue en chlorure d'acide correspondant qui, traité par un alcool ($R_9OH$) ou une amine

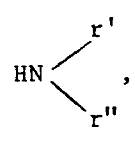

donne l'ester (Ib) ou l'amide (Ic) correspondant.

La présente invention a également pour objet à titre de médicament les composés de formule (I) telle que définie ci-dessus.

Les composés selon l'invention présentent une bonne stabilité à la lumière et à l'oxygène.

Ces composés présentent une bonne activité dans le test de différenciation des cellules de tératocarcinome embryonnaire de la souris (cellules F9) (Cancer Research 43, p 5268, 1983), et/ou dans le test d'inhibition de l'ornithine décarboxylase après induction par "tape stripping" chez le rat nu (Lab. Animals 21, p233-240, 1987) et/ou dans le test de l'oedème de l'oreille induit par application topique d'acide arachidonique chez la souris (J. Invest. Dermatol. 82,p367-371, 1984). Ces tests montrent les activités des composés respectivement dans les domaines de la différenciation, de la prolifération et de l'inflammation.

Enfin, les nouveaux composés se caractérisent par l'introduction, dans la structure chimique, d'une liaison thioester hautement sensible à l'action de diverses estérases in vivo, ce qui conduit à l'inactivation rapide des molécules par conversion en fragments biologiquement inactifs.

Les composés selon l'invention conviennent particulièrement bien dans les domaines de traitement suivants :

1) pour traiter les affections dermatologiques liées à un désordre de la kératinisation portant sur la différenciation et sur la prolifération notamment pour traiter les acnés vulgaires,comédoniennes, polymorphes, les acnés nodulokystiques, conglobata, les acnés séniles, les acnés secondaires telles que l'acné solaire, médicamenteuse, professionnelle ;

2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen ;

3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriasique, ou encore l'atopie cutanée, telle que l'eczéma, ou l'atopie respiratoire ; les composés peuvent également être utilisés dans certaines affections inflammatoires ne présentant pas de trouble de la kératinisation ;

4) pour traiter toutes les proliférations dermiques ou épidermiques qu'elles soient bénignes ou malignes, qu'elles soient d'origine virale telles que les verrues vulgaires, les verrues planes et l'épidermodysplasie verruciforme, les proliférations pouvant également être induites par les ultra-violets notamment dans le cas des épithelioma baso et spino cellulaires ;

5) pour traiter d'autres désordres dermatologiques tels que les dermatoses bulleuses et les maladies du collagène ;

6) pour traiter certains troubles ophtalmologiques, notamment les cornéopathies ;

7) pour lutter contre le vieillissement de la peau, qu'il soit photoinduit ou non ;

8) pour prévenir ou guérir les stigmates de l'atrophie épidermique et/ou dermique induite par les corticostéroïdes locaux ou systémiques, ou toute autre forme d'atrophie cutanée.

La présente invention a donc également pour objet des compositions médicamenteuses contenant au moins un composé de formule (I) tel que défini ci-dessus, ou un de ses sels.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Les composés selon l'invention sont généralement administrés à une dose journalière d'environ 0,01mg/kg à 100mg/kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions ou de nanoparticules. Par voie parentérale, les compositions peuvent se présenter sous forme de solutions, de suspensions ou de nanoparticules pour perfusion ou pour injection.

Par voie topique, les compositions pharmaceutiques à base des composés selon l'invention sont destinées au traitement de la peau et des muqueuses et se présentent sous forme d'onguents, de crèmes, de laits, de pommades, de poudres, de tampons imbibés, de solutions, de gels,de sprays de lotions ou de suspensions. Elles peuvent également se présenter sous forme de microsphères ou vésicules lipidiques ou polymériques ou de patches polymériques ou d'hydrogels permettant une libération contrôlée.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) tel que défini ci-dessus ou un de ses sels, à une concentration de préférence comprise entre 0,0001 et 5% par rapport au poids total de la composition. Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I), dans les compositions cosmétiques est comprise entre 0,0001 et 0,1% en poids et de préférence entre 0,001 et 0,01% en poids.

Les compositions médicamenteuses et cosmétiques selon l'invention peuvent contenir des additifs inertes ou même pharmacodynamiquement ou cosmétiquement actifs et notamment : des agents hydratants comme la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, la tioxolone ou le peroxyde de benzoyle; des antibiotiques comme l'érythromycine et ses esters, la néomycine, les tétracyclines ou les polyméthylène-4,5 isothiazolinones-3; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5-5-diphényl-imidazolidine 2,4-dione); des agents anti-inflammatoires stéroïdiens et non stéroïdiens; des caroténoïdes et, notamment le β-carotène; des agents anti-psoriasiques tels que l'anthraline et ses dérivés et les acides eicosatétraynoïque-5,8,11,14 et triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

EXEMPLE 1

Acide 4-[3-(1-adamantyl)benzoylthio]benzoïque

Dans un ballon, on introduit 843 mg (5,47 mmoles) d'acide 4-mercaptobenzoïque, 9 ml de pyridine et ajoute goutte à goutte une solution de 1,5 g (5,47 mmoles) de chlorure de 3-(1-adamantyl)benzoyle dans 15 ml de dichlorométhane. On agite à température ambiante pendant huit heures, évapore à sec, reprend par l'eau, acidifie à pH 1 avec de l'acide chlorhydrique 1N et extrait avec de l'éther éthylique. On décante la phase organique, lave à l'eau, sèche sur sulfate de magnésium et évapore. Le résidu obtenu est purifié par chromatographie sur colonne de silice, élué avec un mélange de dichlorométhane et d'éther éthylique (80-20). On recueille après évaporation des solvants, 980 mg (45 %)d'acide 4-[3-(1-adamantyl)benzoylthio]benzoïque de point de fusion : 224-226 °C.

EXEMPLE 2

Acide 4-[5-(1-adamantyl)-2-fluoro-4-méthoxybenzoylthio]benzoïque

De manière analogue à l'exemple 1, par réaction de 2,34 g (7,5 mmoles) de chlorure de 5-(1-adamantyl)-2-fluoro-4-méthoxybenzoyle avec 1,15g (7,5 mmoles) d'acide 4-mercaptobenzoïque, on obtient 2,7g (81%) d'acide 4-[5-(1-adamantyl)-2-fluoro-4-méthoxybenzoylthio]benzoïque, de point de fusion : 240-243°C.

EXEMPLE 3

Acide 4-(5,6,7,8-tétrahydro-5,5,8,8-tétraméthyl-2-naphtoylthio)benzoïque

Dans un ballon, on introduit 3,08g (20 mmoles) d'acide 4-mercaptobenzoïque, 20ml de pyridine et ajoute

goutte à goutte une solution de 5g (20 mmoles) de chlorure de 5,6,7,8-tétrahydro-5,5,8,8-tétraméthyl-2-naphtoyle dans 50ml de dichlorométhane. On agite à température ambiante pendant quatre heures, évapore à sec le milieu réactionnel, reprend par l'eau et acidifie à pH 1 avec de l'acide chlorhydrique IN. On extrait avec 700ml d'éther éthylique, décante la phase organique, lave à l'eau, sèche sur sulfate de magnésium et évapore. Le solide obtenu est purifié par chromatographie sur colonne de silice, élué par un mélange de dichlorométhane et d'hexane (90-10). On recueille, après évaporation des solvants, 6g (82%) d'acide 4-(5,6,7,8-tétrahydro-5,5,8,8-tétraméthyl-2-naphtoylthio)benzoïque de point de fusion : 235-237°C.

EXEMPLE 4

Acide 4-[5-(1-adamantyl)-4-méthoxy-2-méthylbenzoylthio]benzoique

a) 5-(1-adamantyl)-4-méthoxy-2-méthylbenzoate de méthyle.

Dans un ballon, on introduit 5,5g (30,5 mmoles) de 4-méthoxy-2-méthylbenzoate de méthyle, 4,7g (30,5 mmoles) de 1-adamantanol et 50ml de dichlorométhane. On ajoute goutte à goutte 1,6ml d'acide sulfurique concentré et agite à température ambiante pendant douze heures. On verse le milieu réactionnel dans l'eau, neutralise avec du bicarbonate de sodium, extrait avec de l'éther éthylique, décante la phase organique, lave à l'eau, sèche sur sulfate de magnésium et évapore. Le résidu obtenu est purifié par chromatographie sur colonne de silice, en éluant avec du dichlorométhane. Après évaporation des solvants, on recueille 6,8g (71%) de 5-(1-adamantyl)-4-méthoxy-2-méthylbenzoate de méthyle de point de fusion : 130-131°C.

b) Acide 5-(1-adamantyl)-4-méthoxy-2-méthylbenzoïque

Une suspension de 6,7g (21 mmoles) de l'ester précédent dans 100ml de soude méthanolique 2N et 100ml de tétrahydrofuranne (THF) est chauffée à reflux pendant quatre heures. Le milieu réactionnel est évaporé à sec, repris par l'eau, acidifié à pH 1 avec de l'acide chlorhydrique concentré et extrait avec de l'éther éthylique. On décante la phase organique, lave à l'eau, sèche sur sulfate de magnésium et évapore. On obtient 6,4g (100%) de l'acide attendu de point de fusion : 244-246°C.

c) Chlorure de 5-(1-adamantyl)-4-méthoxy-2-méthylbenzoyle

2,6g (8,6 mmoles) d'acide 5-(1-adamantyl)-4-méthoxy-2-méthylbenzoïque et 20ml de chlorure de thionyle sont chauffés à reflux jusqu'à cessation du dégagement gazeux. On évapore à sec le milieu réactionnel et obtient 2,7g (100%) du chlorure d'acide brut, utilisé tel quel pour la suite de la synthèse.

d) Acide 4-[5-(1-adamantyl)-4-méthoxy-2-méthylbenzoylthio]benzoïque

De manière analogue à l'exemple 1, par réaction de 2,7g (8,6 mmoles) du chlorure d'acide précédent et de 1,32g (8,6 mmoles) de l'acide 4-mercaptobenzoïque, on obtient 3,1g (84%) d'acide 4-[5-(1-adamantyl)-4-méthoxy-2-méthylbenzoylthio]benzoïque de point de fusion : 264-265°C.

EXEMPLE 5

Acide 4-[5-(1-adamantyl)-2-hydroxy-4-méthoxybenzoylthio]benzoïque

a) 5-(1-adamantyl)-2-hydroxy-4-méthoxybenzoate de méthyle

A une suspension de 3,3g (0,108 mole) d'hydrure de sodium (80% dans l'huile) dans 300ml de diméthylformamide (DMF), on ajoute goutte à goutte 32,5g (0,108 mole) de 5-(1-adamantyl)-2,4-dihydroxybenzoate de méthyle et agite à température ambiante jusqu'à cessation du dégagement gazeux. On ajoute ensuite 7,4ml (0,12 mole) d'iodure de méthyle et agite à température ambiante pendant douze heures. On verse le milieu réactionnel dans l'eau, extrait avec 800ml d'éther éthylique, décante la phase organique, lave à l'eau, sèche sur sulfate de magnésium et évapore. Le résidu obtenu est purifié par chromatographie sur colonne de silice, en éluant avec un mélange de dichlorométhane et d'hexane (30-70). On recueille 18,1g (53%) du produit attendu de point de fusion : 134-135°C.

b) Acide 5-(1-adamantyl)-2-hydroxy-4-méthoxybenzoïque

De manière analogue à l'exemple 4(b), à partir de 4,35g (13,7 mmoles) de l'ester préparé en 5(a), on obtient 3,1g (75%) d'acide 5-(1-adamantyl)-2-hydroxy-4-méthoxybenzoïque de point de fusion : 234-235°C.

c) Acide 4-[5-(1-adamantyl)-2-hydroxy-4-méthoxybenzoylthio]benzoïque.

A une solution de 1,55g (5,13 mmoles) d'acide 5-(1-adamantyl)-2-hydroxy-4-méthoxy benzoïque dans 30ml de toluène anhydre, on ajoute successivement 1,3ml (10,2 mmoles) de chlorotriméthylsilane et 1,45ml (10,3 mmoles) de triéthylamine et chauffe à reflux pendant quatre heures. On filtre le sel formé, évapore le filtrat et dissout le résidu dans 20ml de dichlorométhane. A cette solution, on ajoute 447µl (6,16

mmoles) de chlorure de thionyle et chauffe à reflux pendant une heure. On évapore à sec le milieu réactionnel, ajoute au résidu 20ml de dichlorométhane et additionne cette solution sur un mélange de 790mg (5,13 mmoles) d'acide 4-mercaptobenzoïque dans 10ml de pyridine. On agite à température ambiante pendant huit heures, évapore à sec le milieu réactionnel, reprend par l'eau et acidifie à pH 1 avec de l'acide chlorhydrique IN. On filtre le solide, lave à l'eau, sèche sous vide en présence de pentoxyde de phosphore et recristallise dans un mélange d'éther diisopropylique et d'acétate d'éthyle (1-3). On recueille après séchage, 1,08g (48%) d'acide 4-[5-(1-adamantyl) -2-hydroxy-4-méthoxybenzoylthio]benzoïque de point de fusion : 240-241°C.

EXEMPLE 6

Acide 4-(3,5-di-trifluorométhylbenzoylthio)benzoïque

Dans un ballon, on introduit 400mg (4,92 mmoles) d'acide 4-mercaptobenzoïque, 15ml de pyridine et ajoute goutte à goutte une solution de 1,3g (4,92 mmoles) de chlorure de 3,5-di-trifluorométhylbenzoyle dans 50ml de dichlorométhane. On agite une heure à température ambiante, évapore à sec, reprend par l'eau et acidifie à pH 5 avec de l'acide chlorhydrique IN. Le solide est filtré, lavé à l'eau, séché sous vide en présence de pentoxyde de phosphore puis trituré dans 100ml d'éther éthylique. On recueille 1,7g (88%) d'acide 4-(3,5-di-trifluorométhylbenzoylthio)benzoïque de point de fusion : 162-163°C.

EXEMPLE 7

Acide 4-(3-isopropyl-4-méthoxybenzoylthio)benzoïque

a) 2-isopropylanisole

A une suspension de 3,6g (0,12 mole) d'hydrure de sodium (80% dans l'huile) dans 50ml de THF, on ajoute goutte à goutte une solution de 13,6g (0,1 mole) de 2-isopropylphénol dans 100ml de THF et agite jusqu'à cessation du dégagement gazeux. On additionne ensuite 7,5ml (0,12 mole) d'iodure de méthyle et agite à température ambiante pendant 16 heures. On verse le milieu réactionnel dans l'eau, extrait avec de l'éther éthylique, décante la phase organique, sèche sur sulfate de magnésium et évapore. On recueille 14,3g (95%) de 2-isopropylanisole, sous forme d'une huile légèrement jaune.

b) 3-isopropyl-4-méthoxyacétophénone

Dans un tricol, on introduit 12,7g (96 mmoles) de chlorure d'aluminium et 100ml de dichlorométhane et refroidit à 5°C. On ajoute goutte à goutte un mélange de 14,3g (96 mmoles) de 2-isopropylanisole, 6,78ml (96 mmoles) de chlorure d'acétyle dans 100 ml de dichlorométhane sur une période de trente minutes et laisse remonter la température à 20°C. On verse le milieu réactionnel sur de la glace, extrait avec de l'éther éthylique, décante la phase organique, lave à l'eau, sèche sur sulfate de magnésium et évapore. On recueille 18,2g (100%) du produit attendu brut sous forme d'une huile, qui est utilisé tel quel pour la suite de la synthèse.

c) Acide 3-isopropyl-4-méthoxybenzoïque

A une solution, refroidie à 5°C, de 55,6g (1,39 mmole) d'hydroxyde de sodium dans 250ml d'eau, on ajoute goutte à goutte 18,9ml (0,34mole) de brome et agite 30 minutes en laissant la température remonter à 20°C. On ajoute ensuite, goutte à goutte, une solution de 17,5g (9,15 mmoles) de 3-isopropyl-4-méthoxyacétophénone dans 100ml de dioxanne et agite deux heures à tempéraure ambiante. Le milieu réactionnel est versé dans l'eau, la phase aqueuse est lavée avec de l'éther éthylique, décantée et acidifiée à pH 1 avec de l'acide chlorhydrique concentré. On filtre le solide, et le sèche sous vide en présence de pentoxyde de phosphore. On obtient 15,6g (88%) de l'acide attendu de point de fusion : 164-165°C.

d) Chlorure de 3-isopropyl-4-méthoxybenzoyle

De manière analogue à l'exemple 4(c) à partir de 3,86g (20 mmoles) d'acide 3-isopropyl-4-méthoxybenzoïque, on obtient 4,2g (100%) du chlorure d'acide brut, qui est utilisé tel quel pour la suite de la synthèse.

e) Acide-4-(3-isopropyl-4-méthoxybenzoylthio)benzoïque

De manière analogue à l'exemple 1, par réaction de 4,2g (20 mmoles) du chlorure d'acide précédent et de 3,08g de l'acide 4-mercaptobenzoïque, on obtient 4g (61%) de l'acide 4-(3-isopropyl-4-méthoxybenzoylthio)benzoïque de point de fusion : 224-226°C.

EXEMPLE 8

Acide 4-(3-isopropylthio-4-méthylbenzoylthio)benzoïque

a) 3-isopropylthio-4-méthylbenzoate de méthyle

De manière analogue à l'exemple 7 a), par réaction de 1,6g (8,9 mmoles) de 3-mercapto-4-méthylbenzoate de méthyle avec 890µl (8,9 mmoles) d'iodure d'isopropyle, on obtient 2g (100%) du produit attendu, sous forme d'une huile légèrement jaune.

b) Acide 3-isopropylthio-4-méthylbenzoïque

De manière analogue à l'exemple 4 b), à partir de 1,95g (8,7 mmoles) de l'ester précédent, on obtient 1,8g (100%) d'acide 3-isopropylthio-4-méthylbenzoïque de point de fusion : 122-123°C.

c) Chlorure de 3-isopropylthio-4-méthylbenzoyle

De manière analogue à l'exemple 4(c), à partir de 1,7g (8 mmoles) d'acide 3-isopropylthio-4-méthylbenzoïque, on obtient 1,8g (100%) de chlorure d'acide brut, utilisé tel quel pour la suite de la synthèse.

d) Acide 4-(3-isopropylthio-4-méthylbenzoylthio)benzoïque

De manière analogue à l'exemple 1, par réaction de 1,8g (8 mmoles) du chlorure d'acide précédent avec 1,2g (8 mmoles) d'acide 4-mercaptobenzoïque, on obtient 2,1g (77%) d'acide 4-(3-isopropylthio-4-méthylbenzoylthio)benzoïque de point de fusion : 186-187°C.

EXEMPLE 9

Acide 4-[3-(1-adamantyl)-4-allyloxybenzoylthio]benzoïque

a) 3-(1-adamantyl)-4-allyloxybenzoate de méthyle

De manière analogue à l'exemple 5 a), par réaction de 4,3g (15 mmoles) de 3-(1-adamantyl)-4-hydroxybenzoate de méthyle et de 1,43ml (16,5 mmoles) de bromure d'allyle, on obtient 3,1g (66%) du produit attendu de point de fusion : 69-71°C.

b) Acide 3-(1-adamantyl)-4-allyloxybenzoïque

De manière analogue à l'exemple 4(b), à partir de 3,1g (10 mmoles) de l'ester précédent, on obtient 2,7g (90%) d'acide 3-(1-adamantyl)-4-allyloxybenzoïque de point de fusion : 252-253°C.

c) Chlorure de 3-(1-adamantyl)-4-allyloxybenzoyle

De manière analogue à l'exemple 4 c), à partir de 2,6g (8,6 mmoles) d'acide préparé à l'exemple 9 b), on obtient 2,8g (100%) de chlorure d'acide brut, utilisé tel quel pour la suite de la synthèse.

(d) Acide 4-[3-(1-adamantyl)-4-allyloxybenzoylthio]benzoïque

De manière analogue à l'exemple 1, par réaction de 2,8g (8,6 mmoles) du chlorure d'acide précédent avec 1,32g (8,6 mmoles) d'acide 4-mercaptobenzoïque, on obtient 1,6g (42%) d'acide 4-[3-(1-adamantyl)-4-allyloxybenzoylthio]benzoïque point de fusion : 266-267 °C.

EXEMPLE 10

Acide 4-[3-(1-adamantyl)-4-méthylthiobenzoylthio]benzoïque

a) 3-(1-adamantyl)-4-(N,N-diméthylthiocarbamoyloxy)benzoate de méthyle

A une solution de 22,9g (80 mmoles) de 3-(1-adamantyl)-4-hydroxybenzoate de méthyle dans 100ml de DMF, on ajoute par petites quantités 2,65g (88 mmoles) d'hydrure de sodium (80% dans l'huile) et agite jusqu'à cessation du dégagement gazeux. On ajoute ensuite 15,2g (123 mmoles) de chlorure de N,N-diméthylthiocarbamoyle et agite à 70°C durant 8 heures. On verse le milieu réactionnel dans l'eau, filtre le solide qui a précipité, le lave à l'eau et sèche en présence de pentoxyde de phosphore. On triture le solide dans 70ml d'éther diisopropylique à reflux et obtient après filtration 15g (50%) du produit attendu de point de fusion : 174-175°C.

b) 3-(1-adamantyl)-4-(N,N-diméthylcarbamoylthio)benzoate de méthyle

Dans un ballon et sous courant d'azote, on introduit 15g (37 mmoles) de l'ester précédent et chauffe à 300°C durant 30 minutes. Le résidu obtenu est purifié par chromatographie sur colonne de silice, élué avec du dichlorométhane. Après évaporation des solvants, on recueille 8,7g (62%) de 3-(1-adamantyl)-4-(N,N-diméthylcarbamoylthio)benzoate de méthyle de point de fusion : 150-151°C.

c) Acide 3-(1-adamantyl)-4-mercaptobenzoïque

Une solution de 8,6g (23 mmoles) de l'ester obtenu en 10 b) dans 150ml de soude méthanolique 2N est chauffée à reflux pendant une heure. On évapore à sec, reprend par l'eau, acidifie à pH 1 avec de

l'acide chlorhydrique concentré, extrait avec de l'éther éthylique, décante la phase organique, sèche sur sulfate de magnésium et évapore. Le résidu obtenu est trituré dans le minimum d'éther éthylique puis filtré. On recueille ainsi 5,4g (82%) d'acide 3-(1-adamantyl)-4-mercaptobenzoïque de point de fusion : 235-237°C.

d) Acide 3-(1-adamantyl)-4-méthylthiobenzoïque

A une suspension de 310mg (10,4 mmoles) d'hydrure de sodium (80% dans l'huile) dans 50ml de DMF, on ajoute goutte à goutte 1,5g (5,2 mmoles) d'acide 3-(1-adamantyl)-4-mercaptobenzoïque dissous dans 50ml de DMF et agite jusqu'à cessation du dégagement gazeux. On additionnne ensuite 645µl (5,2 mmoles) d'iodure de méthyle et agite à température ambiante pendant 4 heures, verse le milieu réactionnel dans l'eau, acidifie à pH 4 avec de l'acide chlorhydrique 1N, filtre le solide et le lave à l'eau. Le produit est séché en présence de pentoxyde de phosphore et trituré dans le minimum d'éther éthylique. Après filtration, on obtient 1,3g (75%) d'acide 3-(1-adamantyl)-4-méthylthiobenzoïque de point de fusion : 264-265°C.

e) Chlorure de 3-(1-adamantyl)-4-méthylthiobenzoyle

De manière analogue à l'exemple 4(c), à partir de 1,4g (4,6 mmoles) de l'acide précédent, on obtient 1,5g (100%) de chlorure d'acide brut, utilisé tel quel pour la suite de la synthèse.

f) Acide 4-[3-(1-adamantyl)-4-méthylthiobenzoylthio]benzoïque

De manière analogue à l'exemple 1, par réaction de 1,5g (4,6 mmoles) du chlorure d'acide précédent et de 710mg (4,6 mmoles) d'acide 4-mercaptobenzoïque, on obtient 1,15g (59%) d'acide 4-[3 (1-adamantyl)-4-méthylthiobenzoylthio]benzoïque de point de fusion : 265-267°C.

EXEMPLE 11

3-(1-adamantyl)-4-méthoxythiobenzoate de phényle

Dans un tricol, sous courant d'azote, on introduit 330mg (11mmoles) d'hydrure de sodium (80% dans l'huile) et 20ml de THF. On ajoute goutte à goutte 1,03ml (10mmoles) de thiophénol et agite jusqu'à cessation du dégagement gazeux. On introduit ensuite goutte à goutte une solution de 3,2g (10mmoles) de chlorure de 3-(1-adamantyl)-4-méthoxybenzoyle dans 30ml de THF et agite à température ambiante pendant 18 heures. On verse le milieu réactionnel dans l'eau, extrait avec de l'éther éthylique, lave à l'eau, sèche sur sulfate de magnésium, filtre et évapore. Le résidu est chromatographié sur colonne de silice en éluant avec un mélange de dichlorométhane et d'hexane (50-50). Après évaporation des solvants, on recueille 3,16g (84%) de thioester attendu de point de fusion : 119,5°C.

EXEMPLE 12

3-(1-adamantyl)-4-méthoxythiobenzoate de 4-hydroxyphényle

Dans un tricol, sous courant d'azote, 700mg (5mmoles) de 4-mercaptophénol dans 7ml de THF sont traités par 0,77ml (5,5mmoles) de triéthylamine. On introduit ensuite goutte à goutte une solution de 1,6g (5mmoles)de chlorure de 3-(1-adamantyl)-4-méthoxybenzoyle dans 20ml de THF et agite à température ambiante pendant la nuit. On verse le milieu réactionnel dans l'eau, extrait par de l'éther éthylique, lave à l'eau, sèche sur sulfate de magnésium, filtre et évapore. Le résidu est purifié par chromatographie sur colonne de silice, élué par un mélange d'éther éthylique et d'hexane (50-50). Après évaporation, et précipitation dans l'hexane, on isole 1,82g (92%) du thioester attendu de point de fusion : 239-240°C.

EXEMPLE DE FORMULATION

A. VOIE ORALE

(a) Comprimé de 0,2g

| | |
|---|---|
| Acide 4-(5,6,7,8-tétrahydro-5,5,8,8-tétraméthyl-2-naphtoylthio) benzoïque | 0,001g |
| Amidon | 0,114g |
| Phosphate bicalcique | 0,020g |
| Silice | 0,020g |
| Lactose | 0,030g |
| Talc | 0,010g |
| Stéarate de magnésium | 0,005g |

Dans cet exemple le composé actif peut être remplacé par la même quantité de l'acide 4-[5-(1-adamantyl)-2-hydroxy-4-méthoxybenzoylthio]benzoïque.

(b) Suspension buvable en ampoules de 5 ml

| | |
|---|---|
| Acide 4-[5-(1-adamantyl)-2-fluoro-4-méthoxybenzoylthio] benzoïque | 0,001g |
| Glycérine | 0,500g |
| Sorbitol à 70% | 0,500g |
| Saccharinate de sodium | 0,010g |
| Parahydroxybenzoate de méthyle | 0,040g |
| Arôme qs | |
| Eau purifiée qsp | 5ml |

Dans cet exemple le composé actif peut-être remplacé par la même quantité de l'acide 4-[5-(1-adamantyl)-4-méthoxy-2-méthylbenzoylthio]benzoïque.

(c) Suspension buvable en ampoules de 10ml

| | |
|---|---|
| Acide 4-[3-(1-adamantyl)-4-méthylthiobenzoylthio] benzoïque | 0,200g |
| Glycérine | 1,000g |
| Sorbitol à 70% | 1,000g |
| Saccharinate de sodium | 0,010g |
| Parahydroxybenzoate de méthyle | 0,080g |
| Arôme qs | |
| Eau purifiée qsp | 10ml |

B. VOIE TOPIQUE

(a) Crème H/E anionique

| | | |
|---|---|---|
| Acide 4-(5,6,7,8-tetrahydro-5,5,8,8-tetraméthyl-2-naphtoylthio) benzoïque | | 1,000g |
| Dodecyl sulfate de sodium | | 0,742 g |
| 1,2 propanediol | | 1,540 g |
| Alcool cétylique | | 19,300 g |
| Huile de vaseline épaisse | | 19,300 g |
| Parahydroxybenzoate de méthyle | | 0,075 g |
| Parahydroxybenzoate de propyle | | 0,075 g |
| Eau déminéralisée stérile | qsq | 100,000 g |

(b) Gel éthanolique

| | | |
|---|---|---|
| Acide 4-[5-(1-adamantyl)-2-fluoro-4-méthoxybenzoylthio] benzoïque | | 0,300 g |
| Hydroxypropylcellulose | | 2,000 g |
| Ethanol 95 % | qsq | 100,000 g |

(c) Gel aqueux

| | | |
|---|---|---|
| Acide 4-[5-(1-adamantyl)-2-hydroxy-4-méthoxybenzoylthio] benzoïque | | 0,300g |
| Poloxamer 182 vendu par la Société BASF sous la dénomination "PLURONIC L62" | | 0,200g |
| Propylène glycol | | 4,000g |
| Polymère de l'acide acrylique vendu par la Société Goodrich sous la dénomination "CARBOPOL 940" | | 0,800g |
| EDTA disodique | | 0,100g |
| Parahydroxybenzoate de méthyle | | 0,100g |
| NaOH à 10% dans l'eau | | 1,250g |
| Eau déminéralisée stérile | qsp | 100,000g |

(d) Onguent

| | |
|---|---|
| Acide 4-[5-(1-adamantyl)-4-méthoxy-2-méthylbenzoylthio] benzoïque | 0,020g |
| Myristate d'isopropyle | 81,700g |
| Huile de vaseline fluide | 9,100g |
| Silice vendue par la Société DEGUSSA sous le nom "AEROSIL 200" | 9,180g |

(e) crème H/E non ionique

| | |
|---|---|
| Acide 4-[3-(1-adamantyl)-4-méthylthiobenzoylthio] benzoïque | 1,000g |
| Alcool cétylique | 4,000g |
| Monostéarate de glycérol | 2,500g |
| Stéréate de PEG 50 | 2,500g |
| Beurre de karité | 9,200g |
| Propylène glycol | 2,000g |
| Parahydroxybenzoate de méthyle | 0,075g |
| Parahydroxybenzoate de propyle | 0,075g |
| Eau déminéralisée stérile qsp | 100g |

## Revendications

1. Thioesters bi-aromatiques, caractérisés par le fait qu'ils répondent à la formule générale suivante:

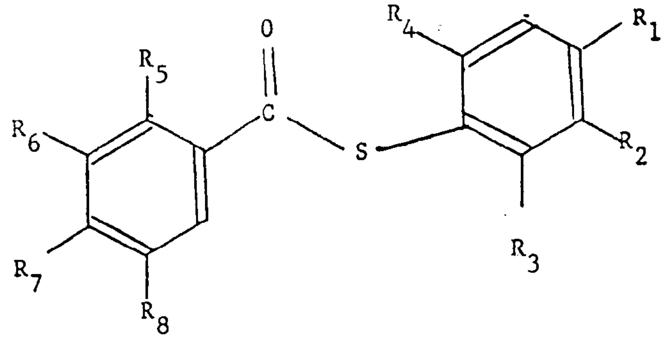

(I)

dans laquelle:

$R_1$ représente un atome d'hydrogène, le groupe OH, le radical $-CH_3$, $-CH_2(OH)$, $-CH(OH)CH_3$, $COOR_9$,

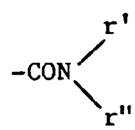

ou $SO_2R_{10}$,

$R_9$ représente un atome d'hydrogène, un radical alkyle ayant de 1 à 6 atomes de carbone ou un radical mono ou polyhydroxyalkyle,

$R_{10}$ représentant le groupe OH, un radical alkyle ayant de 1 à 6 atomes de carbone ou le radical

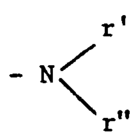

,

r' et r" représentant un atome d'hydrogène, un radical alkyle ayant de 1 à 6 atomes de carbone, aryle, aralkyle, mono ou polyhydroxyalkyle, ou r' et r" pris ensemble forment un hétérocycle,

$R_2$ représente un atome d'hydrogène, un radical alkyle ayant de 1 à 6 atomes de carbone, le radical $OR_9$ , un atome de fluor ou le radical $-CF_3$,

$R_3$ , $R_4$ et $R_5$ représentent un atome d'hydrogène, un atome de fluor, le groupe OH, le radical $-CH_3$ , $-OCH_3$ , $-CF_3$, -COOH ou $CH_2OH$

$R_6$ et $R_6$ représentent un atome d'hydrogène, le radical $-CF_3$, un radical alkyle $\alpha$-substitué ayant de 3 à 15 atomes de carbone, un radical alkyle $\alpha$-$\alpha$'-disubstitué ayant de 4 à 12 atomes de carbone, un radical cycloalkyle ayant de 3 à 12 atomes de carbone, un radical cycloalkyle mono ou polycyclique ayant de 5 à 12 atomes de carbone dont le carbone de liaison est trisubstitué, le radical $-SR_{11}$, $-SO_2R_{11}$ ou $-SOR_{11}$

$R_{11}$ représentant un radical alkyle ayant de 1 à 6 atomes de carbone ou un radical cycloalkyle, $R_6$ et $R_8$ ne pouvant pas simultanément représenter un atome d'hydrogène,

$R_7$ représente un atome d'hydrogène, un radical alkyle ayant de 1 à 6 atomes de carbone, un radical alkényle, un radical alkényloxy, le radical $OR_{12}$ ou $SR_{13}$ ,

$R_{12}$ représentant un atome d'hydrogène, un radical alkyle ayant de 1 à 6 atomes de carbone ou un radical alkényle,

$R_{13}$ représentant un atome d'hydrogène, un radical alkyle ayant de 1 à 6 atomes de carbone ou un radical aralkyle,

$R_6$ et $R_7$ ou $R_7$ et $R_6$ pris ensemble, pouvant également former un cycle à 5 ou 6 chaînons substitué par des groupes méthyle,

sous réserve que lorsque simultanément dans la formule (I) $R_1$ représente $-CH_2OH$, $-CH(OH)CH_3$, $-COOR_9$ ou

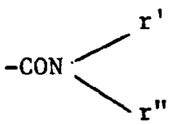

$R_6$ ou $R_6$ représente un radical alkyle $\alpha,\alpha$'-disubstitué ayant de 4 à 12 atomes de carbone ou un radical cycloalkyle mono ou polycyclique ayant de 5 à 12 atomes de carbone dont le carbone de liaison est trisubstitué,

$R_3$ ou $R_4$ représente un atome d'hydrogène ou le radical méthyle et,

$R_7$ représente le radical $OR_{12}$,

alors au moins un des radicaux $R_2$ ou $R_6$ est différent d'un atome d'hydrogène.

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

4. Composés selon la revendication 1, caractérisés par le fait que le radical alkyle $\alpha$-substitué est pris dans le groupe constitué par un radical isopropyle, 1-méthyl propyle, 1-éthyl propyle, 1-méthyl hexyle, 1-méthyl décyle et 1-éthyl dodécyle.

5. Composés selon la revendication 1, caractérisés par le fait que le radical alkyle $\alpha$-$\alpha$'-disubstitué est pris dans le groupe constitué par: un radical tert-butyle, 1,1-diméthyl propyle, 1-méthyl 1-éthyl propyle, 1-méthyl 1-éthyl hexyle et 1,1-diméthyl décyle.

6. Composés selon la revendication 1, caractérisés par le fait que le radical monohydroxyalkyle est un radical 2-hydroxyéthyle, 2-hydroxypropyle ou 3-hydroxypropyle.

7. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxy pentyle et le reste du pentaérythritol.

8. Composés selon la revendication 1, caractérisés par le fait que le radical alkényle ayant de 2 à 6 atomes

de carbone est le radical vinyle, allyle ou 2-butène-yle.

**9.** Composés selon la revendication 1, caractérisés par le fait que le radical aryle est un radical phényle éventuellement substitué par au moins un atome d'halogène, un hydroxyle ou une fonction nitro.

**10.** Composés selon la revendication 1, caractérisés par le fait que le radical aralkyle est le radical benzyle ou le radical phénéthyle éventuellement substitué par au moins un atome d'halogène, un hydroxyle ou une fonction nitro.

**11.** Composés selon la revendication 1, caractérisés par le fait que le radical cycloalkyle mono ou polycyclique ayant de 5 à 12 atomes de carbone dont le carbone de liaison est trisubstitué est le radical 1-méthyl cyclohexyle ou 1-adamantyle.

**12.** Composés selon la revendication 1, caractérisés par le fait que les radicaux r' et r" pris ensemble forment un hétérocycle pris dans le groupe constitué par un radical pipéridino, morpholino, pyrrolidino ou pipérazino éventuellement substitué en position 4 par un radical alkyle en $C_1$-$C_6$ ou mono ou polyhydroxyalkyle.

**13.** Composés selon l'une quelconque des revendications précédentes, caractérisés par le fait qu'ils sont pris dans le groupe constitué par:

Acide 4-[3-(1-adamantyl)benzoylthio]benzoïque,
Acide 4-[5-(1-adamantyl)-2-fluoro-4-méthoxybenzoylthio]benzoïque,
Acide 4-(5,6,7,8-tétrahydro-5,5,8,8-tétraméthyl-2-naphtoylthio)benzoïque,
Acide 4-[5-(1-adamantyl)-4-méthoxy-2-méthylbenzoylthio]benzoïque,
Acide 4-[5-(1-adamantyl)-2-hydroxy-4-méthoxybenzoylthio]benzoïque,
Acide 4-(3,5-di-trifluorométhylbenzoylthio)benzoïque,
Acide 4-(3-isopropyl-4-méthoxybenzoylthio)benzoïque,
Acide 4-(3-isopropylthio-4-méthylbenzoylthio)benzoïque,
Acide 4-[3-(1-adamantyl)-4-allyloxybenzoylthio]benzoïque,
Acide 4-[3-(1-adamantyl)-4-méthylthiobenzoylthio]benzoïque,
3-(1-adamantyl)-4-méthoxythiobenzoate de phényle,
3-(1-adamantyl)-4-méthoxythiobenzoate de 4-hydroxyphényle

**14.** Composés selon l'une quelconque des revendications précédentes caractérisés par le fait qu'ils répondent à la formule générale suivante :

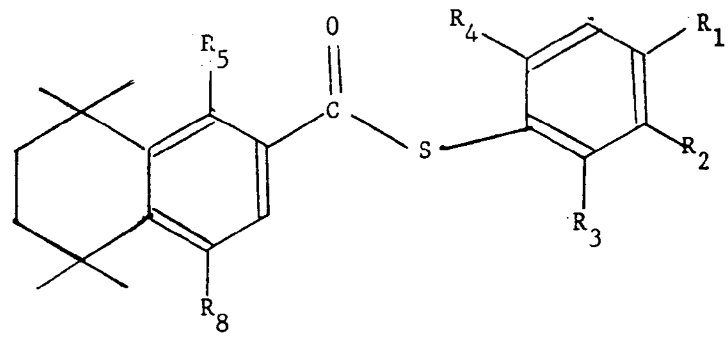

(II)

dans laquelle :
$R_1$ à $R_5$ et $R_8$ sont tels que définis à la revendication 1.

**15.** Composés selon l'une quelconque des revendications 1 à 13 caractérisés par le fait qu'ils répondent à la formule générale suivante :

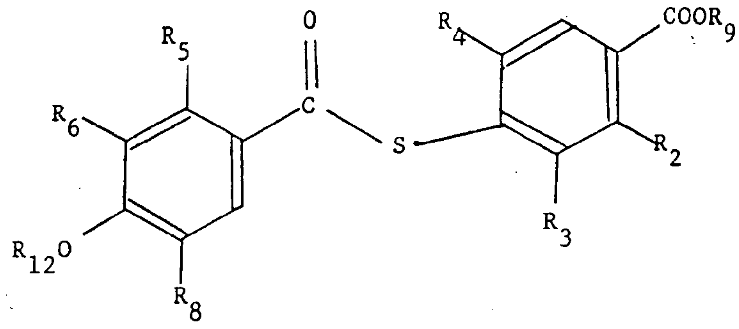

(III)

dans laquelle:

$R_2$ à $R_5$, $R_9$ et $R_{12}$ sont tels que définis ci-dessus à la revendication 1

$R_6$ et $R_8$ représentent un atome d'hydrogène, un radical alkyle $\alpha,\alpha'$-disubstitué ayant de 4 à 12 atomes de carbone ou un radical cycloalkyle mono ou polycyclique ayant de 5 à 12 atomes de carbone dont le carbone de liaison est trisubstitué,

sous réserve que l'un au moins des radicaux $R_6$ ou $R_8$ est différent d'un atome d'hydrogène et que l'un au moins des radicaux $R_2$, $R_4$ ou $R_5$ est différent d'un atome d'hydrogène

**16.** Composés selon l'une quelconque des revendicaitons 1 à 13 caractérisés par le fait qu'ils répondent à la formule générale suivante:

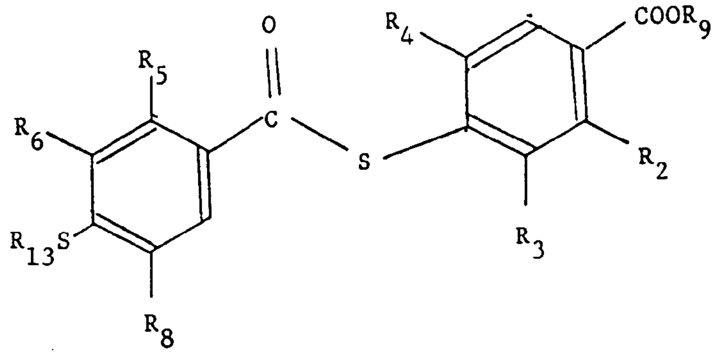

(IV)

dans laquelle :

$R_2$ à $R_5$, $R_8$, $R_9$ et $R_{13}$ sont tels que définis à la revendication 1

**17.** Procédé de préparation des composés de formule (I) selon l'une quelconque des revendications 1 à 16, caractérisé par le fait qu'il consiste à faire réagir, dans un solvant organique, en présence d'une amine tertiaire, une forme activée d'un acide benzoïque substitué notamment un chlorure d'acide de formule:

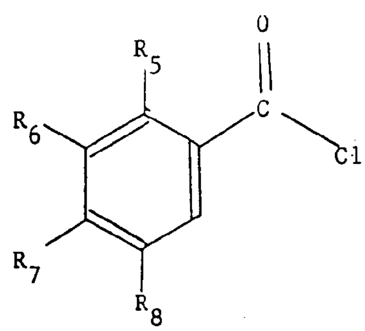

sur un benzènethiol de formule:

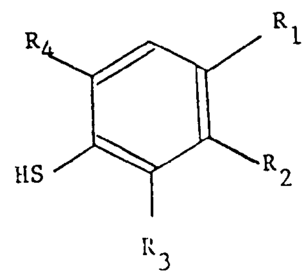

dans lesquelles:

$R_1$ à $R_8$ ont les mêmes significations que celles données à la revendication 1, la réaction étant conduite à température ambiante sous agitation, et que lorsque $R_1$ = -COOH l'on procède si nécessaire à la formation du chlorure d'acide correspondant et à la transformation subséquente dudit chlorure soit en ester par action d'un alcool ($R_9OH$) soit en amide par action d'une amine

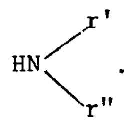

**18.** Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 16.

**19.** Composition selon la revendication 18, caractérisée par le fait qu'elle contient de 0,0001 à environ 5% en poids d'un composé de formule (I).

**20.** Utilisation d'un composé selon l'une quelconque des revendications 1 à 16, pour la préparation d'une composition pharmaceutique destinée au traitement des affections dermatologiques, rhumatismales, respiratoires ainsi qu'ophtalmologiques.

**21.** Composition cosmétique pour l'hygiène corporelle et capillaire, caractérisée par le fait qu'elle contient, dans un véhicule cosmétique approprié, au moins un composé de formule (I) selon l'une quelconque des revendications 1 à 16.

**22.** Composition cosmétique selon la revendication 21, caractérisée par le fait qu'elle contient le composé de formule (I) à une concentration comprise entre 0,0001 et 0,1% et de préférence entre 0,001 et 0,01% en poids.

**Patentansprüche**

**1.** Bi-aromatische Thioester, dadurch gekennzeichnet, daß sie der folgenden allgemeinen Formel entsprechen:

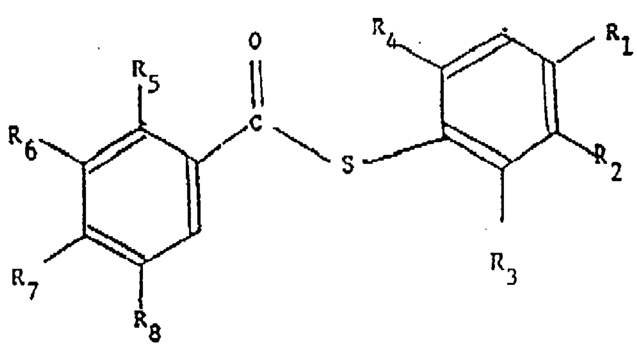

(I)

in der:
$R_1$ ein Wasserstoffatom, die Gruppe OH, den Rest $CH_3$, $-CH_2OH$, $-CH(OH)CH_3$, $-COOR_9$,

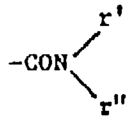

oder $SO_2R_{10}$ bedeutet,
$R_9$ ein Wasserstoffatom, einen Alkylrest mit 1 bis 6 Kohlenstoffatomen oder einen Mono- oder Polyhydroxyalkylrest bedeutet,
$R_{10}$ die Gruppe OH, einen Alkylrest mit 1 bis 6 Kohlenstoffatomen oder den Rest

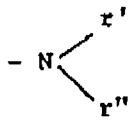

bedeutet, worin r' und r" ein Wasserstoffatom, einen Alkylrest mit 1 bis 6 Kohlenstoffatomen, Aryl, Aralkyl, Mono- oder Polyhydroxyalkyl bedeuten oder r'und r" bilden zusammengenommen einen Heterocyclus,
$R_2$ ein Wasserstoffatom, einen Alkylrest mit 1 bis 6 Kohlenstoffatomen, den Rest $OR_9$, ein Fluoratom oder den Rest $-CF_3$ bedeutet,
$R_3$, $R_4$ und $R_5$ ein Wasserstoffatom, ein Fluoratom, die Gruppe OH, den Rest $-CH_3$, $OCH_3$, $-CF_3$, -COOH oder $-CH_2OH$ bedeuten,
$R_6$ und $R_8$ ein Wasserstoffatom, den Rest $-CF_3$, einen $\alpha$-substituierten Alkylrest mit 3 bis 15 Kohlenstoffatomen, einen $\alpha$-$\alpha'$-disubstituierten Alkylrest mit 4 bis 12 Kohlenstoffatomen, ein Cycloalkylrest mit 3 bis 12 Kohlenstoffatomen, einen mono- oder polycyclischen Cycloalkylrest mit 5 bis 12 Kohlenstoffatomen, worin die Kohlenstoffbindung trisubstituiert ist, den Rest $-SR_{11}$, $-SO_2R_{11}$ oder $-SOR_{11}$ bedeuten, worin
- $R_{11}$ einen Alkylrest mit 1 bis 6 Kohlenstoffatomen oder einen Cycloalkylrest bedeutet, wobei $R_8$ und $R_8$ nicht gleichzeitig ein Wasserstoffatom bedeuten,
$R_7$ ein Wasserstoffatom, einen Alkylrest mit 1 bis 6 Kohlenstoffatomen, einen Alkenylrest, einen Alkenyloxyrest, den Rest $OR_{12}$ oder $SR_{13}$ bedeutet, worin

- $R_{12}$ ein Wasserstoffatom, einen Alkylrest mit 1 bis 6 Kohlenstoffatomen oder einen Alkenylrest bedeutet,
- $R_{13}$ ein Wasserstoffatom, einen Alkylrest mit 1 bis 6 Kohlenstoffatomen oder einen Aralkylrest bedeutet,

$R_6$ und $R_7$ oder $R_7$ und $R_8$ können zusammengenommen ebenfalls einen Ring mit 5 oder 6 Gliedern substituiert durch Methylgruppen bilden, mit der Maßgabe, daß dann, wenn in der Formel (I) gleichzeitig
$R_1$ $-CH_2OH$, $-CH(OH)CH_3$, $-COOR_9$, oder

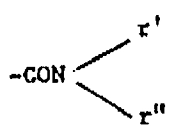

bedeutet,
$R_6$ oder $R_8$ einen $\alpha,\alpha'$-disubstituierten Alkylrest mit 4 bis 12 Kohlenstoffatomen oder einen mono- oder polycyclischen Cycloalkylrest mit 5 bis 12 Kohlenstoffatomen bedeuten, in dem der Kohlenstoff der Bindung trisubstituiert ist, $R_3$ oder $R_4$ ein Wasserstoffatom oder einen Methylrest bedeuten und $R_7$ den Rest $OR_{12}$ bedeutet, muß mindestens einer der Reste $R_2$ oder $R_5$ verschieden von einem Wasserstoffatom sein.

2. Verbindungen nach Anspruch 1, dadurch gekennzeichnet, daß sie in Form eines Alkalimetall- oder eines Erdalkalimetall- oder auch Zinksalzes oder eines organischen Amins vorliegen.

3. Verbindungen nach Anspruch 1, dadurch gekennzeichnet, daß der Niedrigalkylrest mit 1 bis 6 Kohlenstoffatomen aus der Gruppe bestehend aus Methyl, Ethyl, Isopropyl, Butyl und tert.-Butyl stammt.

4. Verbindungen nach Anspruch 1, dadurch gekennzeichnet, daß der α-substituierte Alkylrest aus der Gruppe bestehend aus Isopropyl, 1-Methylpropyl, 1-Ethylpropyl, 1-Methylhexyl, 1-Methyldecyl und 1-Ethyldodecyl stammt.

5. Verbindungen nach Anspruch 1, dadurch gekennzeichnet, daß der α,α'-disubstituierte Alkylrest aus der Gruppe bestehend aus tert.Butyl, 1,1-Dimethylpropyl, 1-Methylethylpropyl, 1-Methyl-1-ethylpropyl, 1-Methyl-1-Ethylhexyl und 1,1-Dimethyldecyl stammt.

6. Verbindungen nach Anspruch 1, dadurch gekennzeichnet daß der Monohydroxyalkylrest ein 2-Hydroxyethylrest, 2-Hydroxypropylrest oder 3-Hydroxypropylrest ist.

7. Verbindungen nach Anspruch 1, dadurch gekennzeichnet, daß der Polyhydroxyalkylrest 3 bis 6 Kohlenstoffatome und 2 bis 5 Hydroxylgruppen aufweist und aus der Gruppe bestehend aus 2,3-Dihydroxypropylrest, 2,3,4-Trihydroxybutyl, 2,3,4,5-Tetrahydroxypentyl und Pentaerythritolrest stammt.

8. Verbindungen nach Anspruch 1, dadurch gekennzeichnet, daß der Alkenylrest 2 bis 6 Kohlenstoffatome aufweist und ein Vinylrest, Allyl- oder 2-Butenylrest ist.

9. Verbindungen nach Anspruch 1, dadurch gekennzeichnet, daß der Arylrest ein gegebenenfalls durch mindestens ein Halogenatom, eine Hydroxylgruppe oder eine Nitrofunktion substituierter Vinylrest ist.

10. Verbindungen nach Anspruch 1, dadurch gekennzeichnet, daß der Aralkylrest, der Benzylrest oder der Phenethylrest gegebenenfalls durch mindestens ein Halogenatom, eine Hydroxylgruppe oder eine Nitrofunktion substituiert ist.

11. Verbindungen nach Anspruch 1, dadurch gekennzeichnet, daß der mono- oder polycyclische Cycloalkylrest 5 bis 12 Kohlenstoffatome aufweist, worin der Bindungskohlenstoff trisubstituiert ist, ein 1-Methylcyclohexyl oder 1-Adamantylrest ist.

12. Verbindungen nach Anspruch 1, dadurch gekennzeichnet, daß die Reste r' und r" zusammengenommen einen Heterocyclus aus der Gruppe Piperidinrest, Morpholinrest, Pyrrolidinrest oder Piperazinrest, gegebenenfalls in Stellung 4 durch einen Alkylrest mit $C_1$ bis $C_6$ oder Mono- oder Polyhydroxyalkylrest substituiert, bilden.

13. Verbindungen nach einem der vorhergehenden Ansprüche, dadurch gekennzeichnet, daß es sich dabei um folgende handelt:
4-[3-(1-Adamantyl)-benzoylthio]-benzoesäure
4-[5-(-1-Adamantyl)-2-fluor-4-methoxybenzoylthio]-benzoesäure
4-[5,6,7,8-Tetrahydro-5,5,8,8-tetramethyl-2-naphtoylthio)-benzoesäure
4-[5-(1-Adamantyl)-2-hydroxy-4-methoxybenzoylthio]-benzoesäure
4-(3,5-Di-Trifluoromethylbenzoylthio)-benzoesäure
4-(3-Isopropyl-4-methoxybenzoylthio)-benzoesäure
4-(3-Isopropylthio-4-methylbenzoylthio)-benzoesäure
4-[3-(1-Adamantyl)-4-allyloxybenzoylthio]-benzoesäure
4-[3-(1-Adamantyl)-4-methylthiobenzoylthio]-benzoesäure
3-Phenyl-(1-adamantyl)-4-methoxythiobenzoat
4-Hydroxyphenyl-3-(1-adamantyl)-4-methoxythiobenzoat.

14. Verbindungen nach einem der vorhergehenden Ansprüche, dadurch gekennzeichnet, daß sie der folgenden allgemeinen Formel entsprechen:

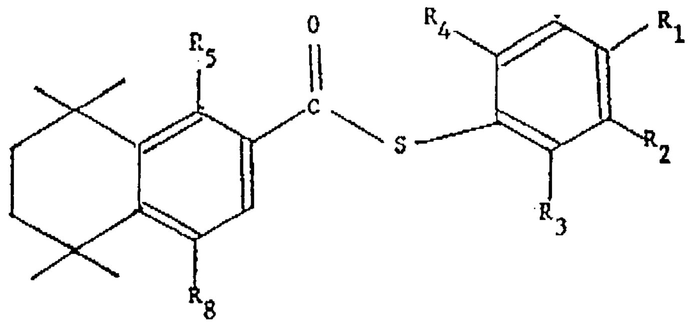

(II)

in der
$R_1$ bis $R_5$ und $R_5$ die in Anspruch 1 gegebene Bedeutung aufweisen.

**15.** Verbindungen nach einem der Ansprüche 1 bis 13, dadurch gekennzeichnet, daß sie der folgenden allgemeinen Formel entsprechen:

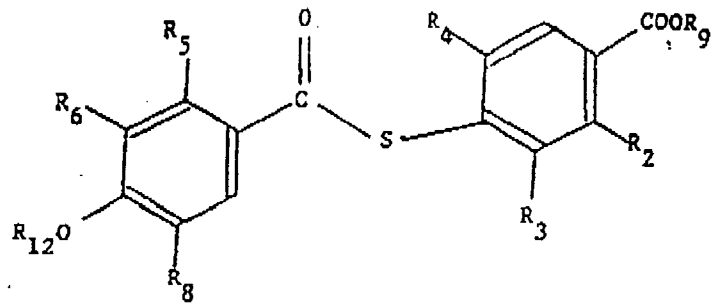

(III)

in der
$R_2$ bis $R_5$, $R_9$ und $R_{12}$ die in Anspruch 1 gegebene Bedeutung aufweisen,
$R_6$ und $R_8$ ein Wasserstoffatom, ein $\alpha,\alpha'$ -disubstituierten Alkylrest mit 4 bis 12 Kohlenstoffatomen oder ein mono- oder polycyclischen Cycloalkylrest mit 5 bis 12 Kohlenstoffatomen, dessen Bindungskohlenstoff trisubstituiert ist, bedeuten, mit der Maßgabe, daß mindestens einer der Reste $R_5$ oder $R_5$ von einem Wasserstoffatom verschieden ist und daß mindestens einer der Reste $R_2$, $R_4$ oder $R_5$ von einem Wasserstoffatom verschieden ist.

**16.** Verbindungen nach einem der Ansprüche 1 bis 13, dadurch gekennzeichnet, daß sie der folgenden allgemeinen Formel entsprechen:

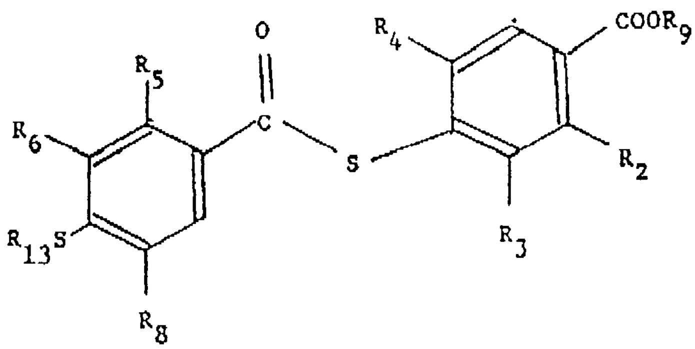

(IV)

in der
$R_2$ bis $R_5$, $R_8$, $R_9$ und $R_{13}$ die in Anspruch 1 gegebene Bedeutung aufweisen.

**17.** Verfahren zur Herstellung der Verbindungen der Formel (I) nach einem der vorhergehenden Ansprüche

1 bis 16, dadurch gekennzeichnet, daß man in einem organischen Lösungsmittel in Gegenwart eines tertiären Amins eine aktivierte Form einer substituierten Benzoesäure, insbesondere ein Säurechlorid, der Formel:

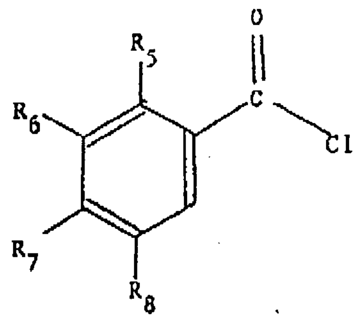

mit einem Benzolthiol der Formel:

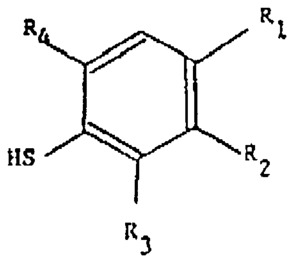

worin:
$R_1$ bis $R_8$ die in Anspruch 1 gegebenen Bedeutungen aufweisen, umsetzt, wobei die Reaktion bei Raumtemperatur unter Rühren durchgeführt wird, und daß man, wenn $R_1$ = -COOH, wenn erforderlich bis zur Bildung des entsprechenden Säurechlorids fortfährt, und anschließend das Chlorid entweder in den Ester durch einen Alkohol ($R_9OH$) oder in ein Amid durch Einwirken eines Amins

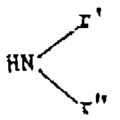

überführt.

**18.** Pharmazeutische Zubereitung, dadurch gekennzeichnet, daß sie in einem für die enterale, parenterale, topische oder oculare Verabreichung geeigneten Trager mindestens eine Verbindung der Formel (I) nach einem der Ansprüche 1 bis 16 enthält.

**19.** Zubereitung nach Anspruch 18, dadurch gekennzeichnet, daß sie 0,0001 bis 5 Gew.-% einer Verbindung der Formel (I) enthält.

**20.** Verwendung einer Verbindung nach einem der Ansprüche 1 bis 16 zur Herstellung einer pharmazeutischen Zubereitung zur Behandlung von dermatologischen, rheumatischen, Atem- und ophtalmologischen Beschwerden.

**21.** Kosmetische Zubereitung zur Körper- und Haarhygiene, dadurch gekennzeichnet, daß sie in einem geeigneten kosmetischen Träger mindestens eine Verbindung der Formel (I) nach einem der Ansprüche 1 bis 16 enthält.

**22.** Kosmetische Zubereitung nach Anspruch 21, dadurch gekennzeichnet, daß sie die Verbindung der Formel (I) in einer Konzentration zwischen 0,0001 und 0,1 Gew.-% und vorzugsweise zwischen 0,001 und 0,01 Gew.-% enthält.

## Claims

1. Biaromatic thioesters, characterized in that they correspond to the following general formula:

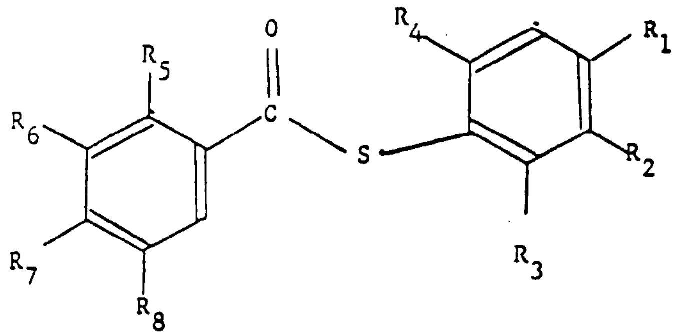

(I)

in which:

$R_1$ represents a hydrogen atom, the OH group or the $-CH_3$, $-CH_2(OH)$, $-CH(OH)CH_3$, $-COOR_9$,

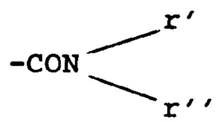

or $SO_2R_{10}$ radical,

$R_9$ represents a hydrogen atom, an alkyl radical having from 1 to 6 carbon atoms or a mono- or polyhydroxyalkyl radical,

$R_{10}$ representing the OH group, an alkyl radical having from 1 to 6 carbon atoms or the

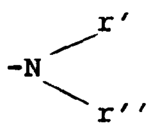

radical,

r' and r" representing a hydrogen atom or an alkyl, having from 1 to 6 carbon atoms, aryl, aralkyl or mono- or polyhydroxyalkyl radical, or r' and r" taken together form a heterocycle,

$R_2$ represents a hydrogen atom, an alkyl radical having from 1 to 6 carbon atoms, the $OR_9$ radical, a fluorine atom or the $-CF_3$ radical,

$R_3$, $R_4$ and $R_5$ represent a hydrogen atom, a fluorine atom, the OH group or the $-CH_3$, $-OCH_3$, $-CF_3$, -COOH or $CH_2OH$ radical,

$R_6$ and $R_8$ represent a hydrogen atom, the $-CF_3$ radical, an α-substituted alkyl radical having from 3 to 15 carbon atoms, an α,α'-disubstituted alkyl radical having from 4 to 12 carbon atoms, a cycloalkyl radical having from 3 to 12 carbon atoms, a mono- or polycyclic cycloalkyl radical having from 5 to 12 carbon atoms, in which the bonding carbon is trisubstituted, or the $-SR_{11}$, $-SO_2R_{11}$ or $-SOR_{11}$ radical

$R_{11}$ representing an alkyl radical having from 1 to 6 carbon atoms or a cycloalkyl radical, it not being possible for $R_6$ and $R_6$ simultaneously to represent a hydrogen atom,

$R_7$ represents a hydrogen atom, an alkyl radical having from 1 to 6 carbon atoms, an alkenyl radical, an alkenyloxy radical or the $OR_{12}$ or $SR_{13}$ radical,

$R_{12}$ representing a hydrogen atom, an alkyl radical having from 1 to 6 carbon atoms or an alkenyl radical,

$R_{13}$ representing a hydrogen atom, an alkyl radical having from 1 to 6 carbon atoms or an aralkyl radical,

$R_6$ and $R_7$ or $R_7$ and $R_8$ taken together also being able to form a 5- or 6-membered ring substituted by methyl groups,

with the proviso that when, simultaneously, in the formula (I)

$R_1$ represents $-CH_2OH$, $-CH(OH)CH_3$, $-COOR_9$ or

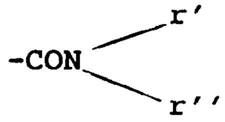

$R_6$ or $R_8$ represents an α,α'-disubstituted alkyl radical having from 4 to 12 carbon atoms or a mono- or polycyclic cycloalkyl radical having from 5 to 12 carbon atoms, in which the bonding carbon is trisubstituted,
$R_3$ or $R_4$ represents a hydrogen atom or the methyl radical and
$R_7$ represents the $OR_{12}$ radical,
then at least one of the $R_2$ or $R_5$ radicals is other than a hydrogen atom.

2. Compounds according to Claim 1, characterized in that they are provided in the form of salts of an alkali metal or alkaline-earth metal or also of zinc or of an organic amine.

3. Compounds according to Claim 1, characterized in that the lower alkyl radical has from 1 to 6 carbon atoms and is taken from the group consisting of the methyl, ethyl, isopropyl, butyl and tert-butyl radicals.

4. Compounds according to Claim 1, characterized in that the α-substituted alkyl radical is taken from the group consisting of an isopropyl, 1-methylpropyl, 1-ethylpropyl, 1-methylhexyl, 1-methyldecyl and 1-ethyldodecyl radical.

5. Compounds according to Claim 1, characterized in that the α,α'-disubstituted alkyl radical is taken from the group consisting of: a tert-butyl, 1,1-dimethylpropyl, 1-methyl-1-ethylpropyl, 1-methyl-1-ethylhexyl and 1,1-dimethyldecyl radical.

6. Compounds according to Claim 1, characterized in that the monohydroxyalkyl radical is a 2-hydroxyethyl, 2-hydroxypropyl or 3-hydroxypropyl radical.

7. Compounds according to Claim 1, characterized in that the polyhydroxyalkyl radical contains from 3 to 6 carbon atoms and from 2 to 5 hydroxyl groups and is taken from the group consisting of the 2,3-dihydroxypropyl, 2,3,4-trihydroxybutyl or 2,3,4,5-tetrahydroxypentyl radical and the pentaerythritol residue.

8. Compounds according to Claim 1, characterized in that the alkenyl radical having from 2 to 6 carbon atoms is the vinyl, allyl or 2-butenyl radical.

9. Compounds according to Claim 1, characterized in that the aryl radical is a phenyl radical optionally substituted by at least one halogen atom, one hydroxyl or one nitro functional group.

10. Compounds according to Claim 1, characterized in that the aralkyl radical is the benzyl radical or the phenethyl radical optionally substituted by at least one halogen atom, one hydroxyl or one nitro functional group.

11. Compounds according to Claim 1, characterized in that the mono- or polycyclic cycloalkyl radical having from 5 to 12 carbon atoms in which the bonding carbon is trisubstituted is the 1-methylcyclohexyl or 1-adamantyl radical.

12. Compounds according to Claim 1, characterized in that the r' and r" radicals, taken together, form a heterocycle taken from the group consisting of a piperidino, morpholino, pyrrolidino or piperazino radical, the piperazino radical optionally being substituted in the 4-position by a $C_1$-$C_6$ alkyl or mono- or polyhydroxyalkyl radical.

13. Compounds according to any one of the preceding claims, characterized in that they are taken from the group consisting of:
4-[3-(1-Adamantyl)benzoylthio]benzoic acid,
4-[5-(1-Adamantyl)-2-fluoro-4-methoxybenzoylthio]benzoic acid,
4-[5,6,7,8-Tetrahydro-5,5,8,8-tetramethyl-2-naphthoylthio)benzoic acid,
4-[5-(1-Adamantyl)-4-methoxy-2-methylbenzoylthio]benzoic acid,
4-[5-(1-Adamantyl)-2-hydroxy-4-methoxybenzoylthio]benzoic acid,

4-[3,5-Di(trifluoromethyl)benzoylthio]benzoic acid,
4-(3-Isopropyl-4-methoxybenzoylthio)benzoic acid,
4-(3-Isopropylthio-4-methylbenzoylthio)benzoic acid,
4-[3-(1-Adamantyl)-4-allyloxybenzoylthio]benzoic acid,
4-[3-(1-Adamantyl)-4-methylthiobenzoylthio]benzoic acid,
Phenyl 3-(1-adamantyl)-4-methoxythiobenzoate,
4-Hydroxyphenyl 3-(1-adamantyl)-4-methoxythiobenzoate.

**14.** Compounds according to any one of the preceding claims, characterized in that they correspond to the following general formula:

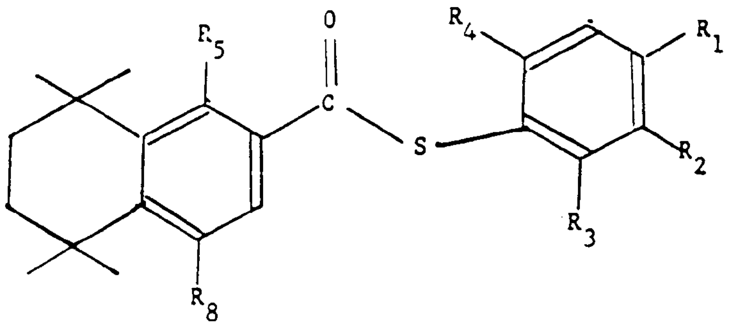

(II)

in which:
$R_1$ to $R_5$ and $R_5$ are as defined in Claim 1.

**15.** Compounds according to any one of Claims 1 to 13, characterized in that they correspond to the following general formula:

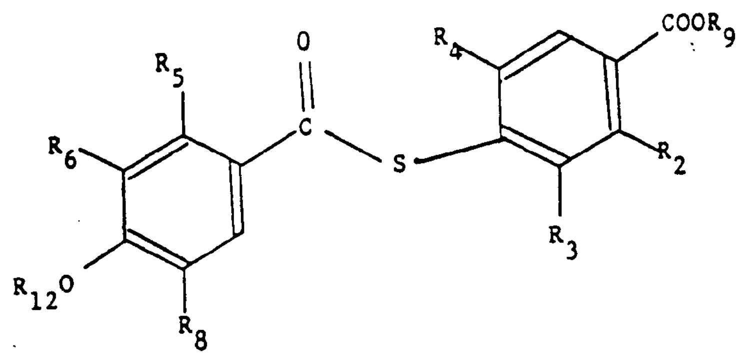

(III)

in which:
$R_2$ to $R_5$, $R_9$ and $R_{12}$ are as defined above in Claim 1
$R_6$ and $R_5$ represent a hydrogen atom, an $\alpha,\alpha'$-disubstituted alkyl radical having from 4 to 12 carbon atoms or a mono- or polycyclic cycloalkyl radical having from 5 to 12 carbon atoms in which the bonding carbon is trisubstituted,
with the proviso that at least one of the $R_5$ or $R_8$ radicals is other than a hydrogen atom and that at least one of the $R_2$, $R_4$ or $R_5$ radicals is other than a hydrogen atom.

**16.** Compounds according to any one of Claims 1 to 13, characterized in that they correspond to the following general formula:

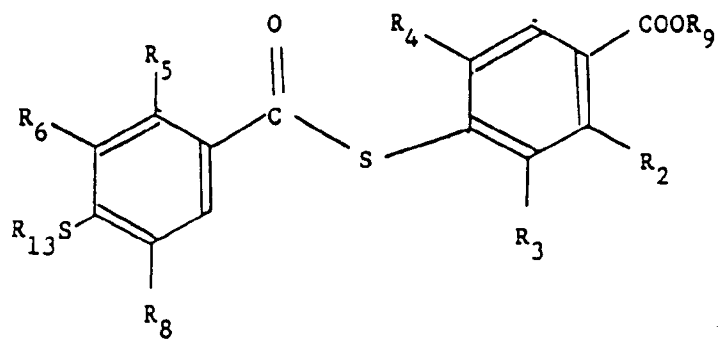

(IV)

in which:

$R_2$ to $R_6$, $R_6$, $R_9$ and $R_{13}$ are as defined in Claim 1.

**17.** Process for the preparation of the compounds of formula (I) according to any one of Claims 1 to 16, characterized in that it consists in reacting, in an organic solvent, in the presence of a tertiary amine, an activated form of a substituted benzoic acid, especially an acid chloride of formula:

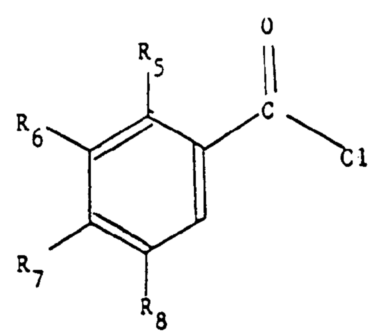

with a benzenethiol of formula:

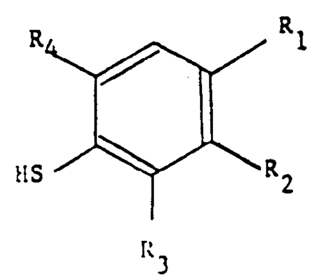

in which:

$R_1$ to $R_8$ have the same meanings as those given in Claim 1, the reaction being carried out at room temperature with stirring, and in that, when $R_1$ = -COOH, the formation of the corresponding acid chloride and the subsequent conversion of the said chloride either to an ester, by reacting with an alcohol ($R_9OH$), or to an amide,
by reacting with an amine

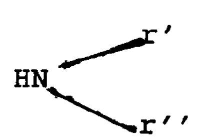

,

are carried out, if necessary.

**18.** Pharmaceutical composition, characterized in that it contains, in a suitable vehicle, for enteral, parenteral,

topical or ocular administration, at least one compound of formula (I) according to any one of Claims 1 to 16.

**19.** Composition according to Claim 18, characterized in that it contains from 0.0001 to approximately 5 % by weight of a compound of formula (I).

**20.** Use of a compound according to any one of Claims 1 to 16 for the preparation of a pharmaceutical composition intended for the treatment of dermatological, rheumatic, respiratory and ophthalmological ailments.

**21.** Cosmetic composition for body and hair hygiene, characterized in that it contains, in a suitable cosmetic vehicle, at least one compound of formula (I) according to any one of Claims 1 to 16.

**22.** Cosmetic composition according to Claim 21, characterized in that it contains the compound of formula (I) at a concentration between 0.0001 and 0.1 % and preferably between 0.001 and 0.01 % by weight.